# EUROPEAN PATENT SPECIFICATION

(11) **EP 2 316 495 B1**
(45) Date of publication and mention of the grant of the patent: **30.05.2012**
(21) Application number: 10189012.7
(22) Date of filing: 27.10.2010
(51) Int. Cl.: A61L 2/08, B65B 55/02

(54) **Electron beam sterilizer**
Elektronenstrahlensterilisator
Stérilisateur de faisceaux électriques

(30) Priority: 29.10.2009 JP 2009249107
(43) Date of publication of application: 04.05.2011
(73) Proprietor: SHIBUYA KOGYO CO., LTD, Kanazawa-shi Ishikawa 920-8681 (JP)
(72) Inventor: Nishino, Yukinobu, Ishikawa 920-8681 (JP); Hayashi, Masami, Ishikawa 920-8681 (JP); Susaki, Shigeru, Ishikawa 920-8681 (JP); Abe, Ryo, Ishikawa 920-8681 (JP)
(74) Representative: HOFFMANN EITLE

(56) References cited:
- WO-A1-2008/146654
- WO-A2-2004/061890
- JP-A- 2007 126 168
- US-A1- 2007 114 433

## Description

### BACKGROUND OF THE INVENTION AND RELATED ART

The present invention relates to an apparatus for sterilizing a vessel, now being conveyed, by being irradiated with electron beam, and more particularly, to an electron beam sterilizer provided with a structure for detecting an electron beam irradiation amount in a case when the electron beam irradiation amount exceeds a predetermined reference value.

In a conventional technology, there is well known an apparatus for irradiating a vessel made of resin such as PET bottle, during conveyance thereof, with electron beam irradiated from an electron beam irradiating apparatus (electron beam irradiator). In such electron beam irradiator, in a case if an electron beam irradiation amount is reduced by any reason, for example, generation of spark within the electron beam irradiator, there causes an improper irradiation to the vessel, which may result in insufficient sterilization of a resin bottle. If such case is caused and a vessel which is insufficiently irradiated with the electron beam is provided, it is required for such vessel to be rejected outside a vessel conveyance and production line before carrying out a subsequent process such as liquid filling process. In order to obviate such defective matter, an electron beam sterilizer which can detect a case where electron beam irradiating amount to the vessel is reduced has been provided, for example, as disclosed in Japanese Granted Patent Publication No. 4148391 (Patent Document 1) or Japanese Laid-open Patent Publication No. 2007-126171 (Patent Document 2).

A sterilizing apparatus disclosed in the Patent Document 1 is provided with a radiation irradiating unit for irradiating plastic vessels (bottles) with radiation, a measuring unit for measuring a light transmission (rate) or optical reflection (rate) to the plastic vessels which are irradiated with the radiation by the radiation irradiating unit, a judging unit for judging degree of sterilization of the plastic vessel based on the light transmission or optical reflection measured by the measuring unit, and a vessel selecting unit for selecting the plastic vessels in accordance with the result judged by the judging unit, to thereby select vessels (bottles) to which the radiation irradiated amount is insufficient and then reject such vessels from the line.

Further, an electron beam sterilizing and inspecting system for food containers or like disclosed in the Patent Document 2 is provided with a food container conveying unit for conveying the food containers, electron beam irradiation unit for irradiating the food container with the electron beam, a physicality (physical property) detecting unit for detecting at least one physical property (such as temperature, ozone density, charged amount, color and the like) changed by irradiating the food containers with the electron beam from the electron beam irradiation unit, and a physical property judging unit for judging whether the physical property detected by the physicality detecting unit or the changing amount of the physical property changed before and after the electron beam irradiation is within a preliminarily set range or not.

Furthermore, other than the inventions disclosed in the above two prior Patent Documents 1 and 2, there is further provided a technology for measuring irradiating amount of the electron beam in term of an electric current as disclosed in Japanese Laid-open Patent Publication HEI 11-248893 (Patent Document 3).

The electron beam irradiation apparatus disclosed in the Patent Document 3 is provided with a rod-shaped collector electrode disposed outside an electron beam irradiation window of an electron beam accelerator and along a short side portion of the irradiation window, a driving mechanism for moving the collector electrode in a direction along a long side portion of the irradiation window in parallel therewith within an electron beam irradiation area, and an electric current measuring unit for measuring an electric current flowing the collector electrode. The collector electrode is electrically insulated from an earth by insulating members disposed at both end portions thereof.

The inventions disclosed in the above Patent Documents 1 and 2 both relate to an apparatus for inspecting vessels after the irradiation with the electron beam. In an electron beam sterilizer, since a transformed X-ray is caused at a portion near the electron beam irradiating position, there may cause a case in which an electronic device such as a measuring equipment, a camera or the like utilized for the measurement or inspection is erroneously operated, and accordingly, it is difficult to dispose such a device as the measuring equipment or camera inside the electron beam sterilizer which is shielded. Because of this reason, these devices are arranged, to carry out the inspection, at portions apart from the position at which the vessels are irradiated with the electron beam. In such arrangement or structure, if there causes a case in which the vessel is insufficiently sterilized because of insufficiently reduced electron beam irradiation, such vessel stays for a long time within the electron beam sterilizer or on a vessel conveying paths for performing subsequent steps or processes such as by a filling unit or capping unit disposed downstream side of the electron beam sterilizer, which may provide an inconvenient matter such as of increasing a risk for scattering virus or bacteria adhering to such vessel into an environmental atmosphere. Moreover, if such insufficiently sterilized vessels are continuously provided, finding of such defective matter may be delayed and many insufficiently sterilized vessels may be provided, thus providing a serious matter.

Still furthermore, since the vessel sterilized by the irradiation with the electron beam is transferred to be subjected to the succeeding downstream-side processes, such as filling process or capping process, with aseptic condition being maintained, it is necessary to maintain an environmental atmosphere through which the sterilized vessels pass with the aseptic condition. For this reason, it is required for equipments and the like utilized for the measurement or inspection to be provided with resistance property against a sterilizing agent for decontaminating the environmental atmosphere.

Still furthermore, in the invention disclosed in the Patent Document 3, a collector is disposed outside an irradiation window through which the electron beam is emitted and is moved in front thereof, and accordingly, it is difficult to measure the electric current during the sterilization operation of the electron beam sterilizer, and hence, it is impossible to immediately detect shortage of the electron beam irradiation amount and then reject the defective vessels from the line.

Reference is also made to JP 2007 126168 A and to WO 2004/06 1890 A2. The two-part form adopted in claim 1 is based on JP 2007 126168 A.

### SUMMARY OF THE INVENTION

The invention is defined by independent claim 1 below. Dependent claims are directed to optional features and preferred embodiments.

The present invention was conceived to solve or improve the defective matters encountered in the conventional technology mentioned above and embodiments of the present invention can provide an electron beam sterilizer in which when a defective vessel due to improper electron beam irradiation is generated, such vessel can be found and then rejected in an early stage of an operation to thereby reduce a fear of introducing contaminated substance such as virus or bacteria into a downstream side environment.

Embodiments of the present invention can also provide an electron beam sterilizer capable of preventing generation of a lot of defective vessels by finding in an early stage where the defective vessel is generated.

The nature and further characteristic features of the present invention will be made clearer from the following descriptions made with reference to the accompanying drawings.

### BRIEF DESCRIPTION OF THE DRAWINGS

To enable a better understanding of the present invention, and to show how the same may be carried into effect, reference will now be made, by way of example only, to the accompanying drawings, in which:-
Fig. 1 is a plan view illustrating an entire arrangement of an electron beam sterilizer according to a first embodiment of the present invention;
Fig. 2 is a perspective view showing an essential portion, in an enlarged scale, of the electron beam sterilizer according to the first embodiment;
Fig. 3 is a graph representing a case of measuring an electric current passing through an electric current measuring electrode and then judging whether an electron beam irradiating amount is proper or improper;
Fig. 4 is graph representing another case of measuring an electric current passing through an electric current measuring electrode and then judging whether an electron beam irradiating amount is proper or improper; and
Fig. 5 is a plan view illustrating an entire arrangement of an electron beam sterilize according to a second embodiment of the present invention.

### DETAILED DESCRIPTION

The present invention which may achieve the above advantages provides a structure, in which a vessel is conveyed, by a vessel conveying unit in front of an electron beam irradiation window of an electron beam irradiator which irradiates the vessel with electron beam, and the vessel is sterilized by the electron beam irradiated through the irradiation window. An electric current measuring electrode is disposed on a side opposite to the irradiation window with a vessel conveyance path interposed therebetween so as to face the irradiation window, and an electric current measuring unit is connected to a lead for grounding the electric current measuring electrode.

When the vessel now being conveyed is irradiated with the electron beam through the irradiation window, the electron beam irradiates the current measuring electrode to thereby pass an electric current. This electric current is measured by the current measuring unit and the measured result is sent to a control device.

The control device includes a comparing unit, a judging unit and a command (instructing) unit, in which the measured current is compared with a preliminarily stored current value by the comparing unit, and based on this comparison, the judging unit judges whether the irradiating amount of the electron beam is proper or not. A rejecting unit is further provided on the downstream side of the electron beam irradiation position so as to extract and then reject a vessel from the vessel conveyance path, and in an occurrence of an event in which it is judged, by the judging unit, that the vessel is not properly irradiated with the electron beam, the command unit instructs to the rejecting unit such that that vessel should be rejected. According to such structural arrangement, the fact that the vessel is properly or improperly irradiated with the electron beam at the irradiation position can be detected, and the defective vessel can be immediately removed from the production line.

A preferred embodiment of the present invention will be explained more clearly hereunder with reference to the accompanying drawings.

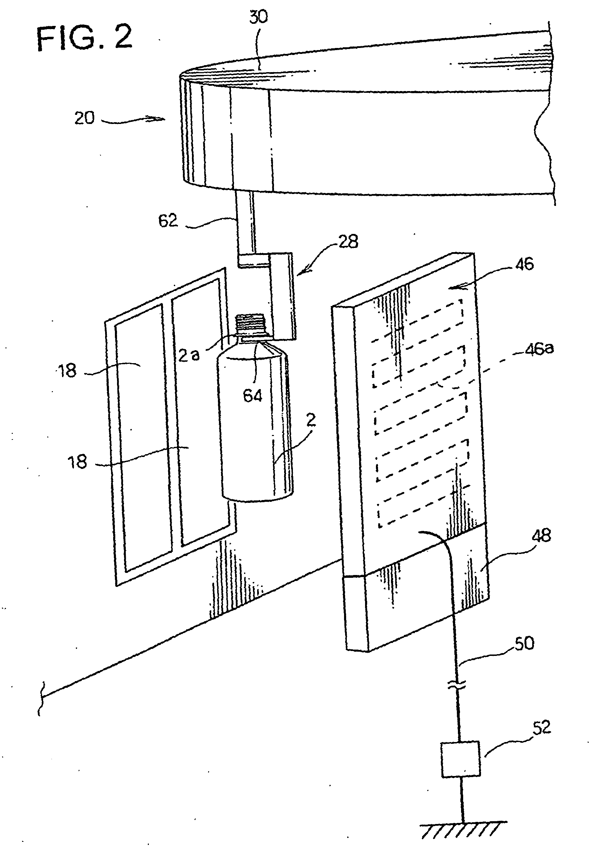

A vessel 2, which is sterilized by an electron beam sterilizer according to this embodiment and filled up with an inner content such as liquid in the subsequent stage or process, is a resin bottle such as PET bottle (which will be explained in detail hereinafter with reference to Fig. 2).

This resin bottle 2 is supported at a lower surface side of a flanged portion 2a formed to a neck portion of the bottle by a support rail of an air conveyer, not shown. The supported bottle is blown with air from a back side thereof and is continuously conveyed to the electron beam sterilizer. The conveyed resin bottle 2 is carried into an introduction chamber 4 and transferred to a carry-in wheel 6 disposed inside the introduction chamber 4.

The carry-in wheel 6 disposed inside the introduction chamber 4 is provided with a plurality of vessel holding means (vessel holders) 8 with equal interval along a circumferential direction of an outer periphery of a rotary body, and the carry-in wheel 6 receives the resin bottle 2 transferred from the air conveyer disposed on the upstream side thereof and conveys downwardly while rotating.

Continuous to the introduction chamber 4, a shield chamber 10 constructed by a lead wall which shields the electron beam or X-ray (braking X-ray) so as not to leak outside at a time when the resin bottle 2 is sterilized by the electron beam irradiation. The interior of the shield chamber 10 is defined into several sections including a supply chamber section 14 which is disposed on an inlet side of the shield chamber 10 and in which a supply wheel 12 is arranged, a main chamber section 22 in which a rotary-type vessel conveyer (i.e., bottle conveyer) 20 for moving the resin bottle 2 received and conveyed from the supply wheel 12 in front of an electron beam irradiation window 18 of an electron beam irradiator (electron beam irradiating device) 16, which will be described hereinafter, and a discharge chamber section 26 in which a discharge wheel 24 is disposed for receiving and discharging the resin bottle 2 sterilized by the electron beam irradiation from the electron beam irradiator 16.

The shield chamber 10 is formed with an opening port 16a formed in the wall section thereof at a portion at which the resin bottle 2 is transferred from the carry-in wheel 6 of the introduction chamber 4 to the supply wheel 12 disposed in the supply chamber 14. The supply wheel 12 receiving the resin bottle 2 from the carry-in wheel 6 of the introduction chamber 4 transfers the resin bottle 2 to a bottle conveyer 20 disposed within the main chamber 22. An opening port, not shown, through which the resin bottle 2 can be transferred, is formed in a partition wall section 14a disposed between the supply chamber 14 and the main chamber 22.

A plurality of grippers 28 (described in detail hereinafter with reference to Fig. 2) as vessel holding means 8 are provided at equal interval in the circumferential direction of the outer periphery of the rotary body 30 in the bottle conveyer 20 disposed within the main chamber 22. Further, a plurality of vessel holding means (holders) 32 are provided for the supply wheel 12, which transfers the bottles to the grippers 28 of the bottle conveyer 20, at equal interval in the circumferential direction of the outer periphery of the rotary body.

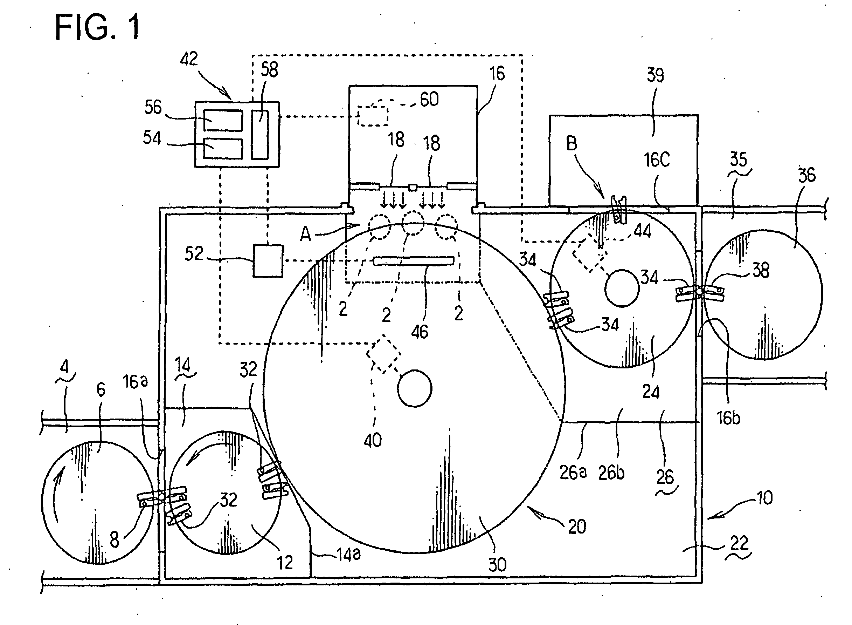

The electron beam irradiating means (electron beam irradiator) 16 is disposed in adjacent to a side wall section (upper side wall in Fig. 1) of a shield chamber 10 made of lead. The electron beam irradiator 16 serves, as is known, to heat filaments in a vacuum atmosphere condition in a vacuum chamber to thereby generate thermal electrons, accelerate the electrons by applying high voltage to generate high speed electron beam, take out the electron beam into atmosphere through a window foil, made of metal such as Ti, attached to the electron beam irradiation window 18 , and then irradiates, with the electron beam, an article to be irradiated (i.e.. resin bottle 2 in this embodiment) positioned within an electron beam irradiation zone or area A in front of the irradiation window 18, thereby performing the sterilization and the like process.

An area in front of the irradiation window 18 of the electron beam irradiator 16 is formed as the electron beam irradiation zone A in which the resin bottle 2 is irradiated with the electron beam.

The discharge chamber 26 is defined by a wall section 26a and a ceiling section 26b from a position near the portion through which the resin bottle 2 conveyed by the bottle conveyer 20 passes the electron beam irradiation zone A. The resin bottle 2 irradiated with the electron beam in the electron beam irradiation zone A is transferred to the discharge wheel 24 disposed inside the discharge chamber 26 from the gripper 28 formed to the bottle conveyer 20. The discharge wheel 24 is provided with a plurality of vessel holders 34 at equal interval in the circumferential direction of the outer periphery of the rotary body, hence, the resin bottle 2 held by each gripper 28 is taken out by the vessel holder 34 and then discharged outward.

The discharge wheel 24 disposed in the discharge chamber 26 also serves as a reject wheel, and when it is judged that the resin bottle 2 is normally and properly sterilized, the resin bottle 2 received from the bottle conveyer 20 is transferred to the vessel holder 38 attached to the carry-out wheel 36 provided for the subsequent intermediate chamber 35, and then, transferred downward so as to be subjected to succeeding processes such as filling process, capping process and so on. These processes will be explained hereinafter.

At a portion in which the resin bottle 2 is transferred from the discharge wheel 24 in the discharge chamber 26 to the carry-out wheel 36 in the intermediate chamber 35, an opening 16b is formed in the wall section of the shield chamber 10 and which has a size through which the resin bottle 2 can be transferred to the carry-out wheel 36.

On the other hand, at a time when it is judged that the sterilization is insufficiently performed by, for example, shortage of the electron beam irradiation amount, the resin bottle 2 is not transferred to the carry-out wheel 36 in the intermediate chamber 35 and discharged to the rejecting section 39 disposed adjacent to the shield chamber 10. Reference character B shown in Fig. 1 represents a rejecting position.

Further, another opening 16c is also formed to the wall section of the shield chamber 10 at a position through which the resin bottle 2 is discharged from the discharge wheel 24 of the discharge chamber 26 to the rejecting section 39. This opening 16c also has a size through which the resin bottle 2 can pass.

The bottle conveyer 20 is provided with an encoder 40, from which a pulse signal is transmitted to the control device 42 so as to always detect the rotating position of the rotary body 30 of the bottle conveyer 20, that is, a position of the resin bottle 2 held by each gripper 28.

Furthermore, another encoder 44 is also provided with the discharge wheel 24 disposed downstream side of the bottle conveyer 20, from which a pulse signal is transmitted to the control device 42 so as to always detect the position of the resin bottle 2, which is transferred from the gripper 28, held by the vessel holder 34. Accordingly, as explained hereinafter, in the case of the shortage or excess of the electron beam irradiation amount from the electron beam irradiator 16, a resin bottle 2 to which the electron beam is insufficiently irradiated is specified by the signal from the encoder 40 of the bottle conveyer 20, and then, the specified resin bottle 2 is traced by the pulse signal from the encoder 44 of the reject wheel (discharge wheel) 24, thereby taking out the specified resin bottle 2 from the reject wheel 24.

A beam collector 46 serving as an electric current measuring electrode is disposed in a vertically standing attitude in front of the electron beam irradiation window 18 of the electron beam irradiator 16 with the vessel conveying path of the bottle conveyer 20 interposed therebetween. A space defined between the electron beam irradiation window 18 and the beam collector 46 is formed as the electron beam irradiation zone A, in which the resin bottle 2 held by the gripper 28 is irradiated with the electron beam at a period when the resin bottle 2 passes the zone A at an approximately intermediate position between the irradiation window 18 and the beam collector 46.

The beam collector 46 is generally formed of a conductive material such as stainless, has a size capable of covering the entire front surface area of the irradiation window 18 and is disposed in front of the radiation window so as to oppose the front surface thereof.

Further, in this embodiment, the irradiation window 18 is composed of divided two parts in the vessel conveying direction, and the beam collector 46 has a size larger than the entire vertical and horizontal sizes of the two parts of the entire irradiation window 18.

The beam collector 46 is provided inside with a cooling water pipe line 46a so as to cool the beam collector 46 to prevent the beam collector from overheating by the electron beam irradiation, and cooling water flows inside the cooling water pipe line 46a.

As shown in Fig. 2, the beam collector 46 is placed on a floor of the main chamber 22 inside the shield chamber 10 via a support member 48 formed of an insulating material in a state supported by the electron beam sterilizer in an electrically insulated manner. An electric current measuring device 52 is connected to a lead 50 grounding the beam collector 46 so as to measure the electric current passing through the earth from the beam collector 46 at the irradiation with the electron beam. The electric current detected by the current measuring device 52 is input into the control device 42.

The current value sent to the control device 42 from the current measuring device 52 is compared with a predetermined reference value in a comparator 54. Based on the compared result by the comparator 54, a judging unit 56 judges whether the electron beam irradiation amount from the electron beam irradiator 16 is proper or not (i.e. improper). In a case of improper amount in the judgement of the judging unit 56, a defective resin bottle is taken out and rejected by the reject wheel (discharge wheel) 24 in response to the instructions from a command unit 58.

Further, the electron beam irradiator 16 is provided with an electric current monitor 60 as electric current recognition unit so as to monitor an output value of the current at all times. The comparator 54 serves to change the reference value in accordance with the variation of the supply current to the electron beam irradiator 16 recognized by the current monitor 60.

The electron beam sterilizer of the structures mentioned above will operate and function as follows.

The resin bottle 2 conveyed by an air conveyer, not shown, enters the introduction chamber 4 and is transferred to the vessel holder 8 of the carry-in wheel 6. The resin bottle 2 is then conveyed while rotating by the carry-in wheel 6 and transferred to the supply wheel 12 disposed in the supply chamber 14 within the shield chamber 10 made of lead. Thereafter, the resin bottle 2 is rotated and conveyed in the state held by the vessel holder 32 of the supply wheel 12 and then transferred to the gripper 28 of the bottle conveyer 20 disposed in the main chamber 22. Although the gripper 28 having various structures may be used, the gripper 28 in this embodiment has a gripping portion 64, as shown in Fig. 2, formed to the lower end of the rotary support shaft 62 extending vertically in a manner such that the gripping portion 64 can hold the lower side of the flanged portion 2a of the resin bottle 2.

The bottle 2 rotated and conveyed in accordance with the rotation of the rotary body 30 in a state held by the gripper 28 of the bottle conveyer 20 reaches the electron beam irradiation zone A positioned in front of the electron beam irradiation window 18 of the electron beam irradiator 16. Within the irradiation zone A. the electron beam is emitted through the irradiation window 18 of the electron beam irradiator 16 to thereby irradiate the resin bottles 2, with the electron beam, which are held by the grippers 28 arranged at predetermined interval to the bottle conveyer 20.

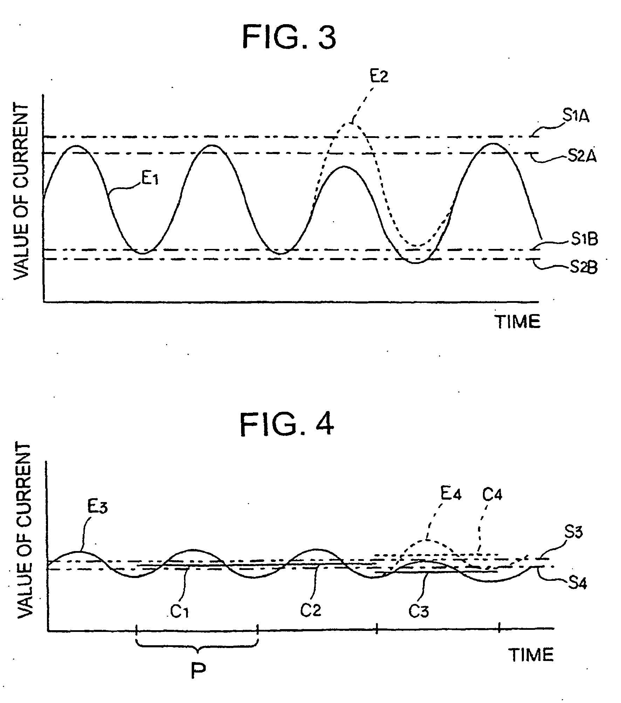

The electron beam emitted toward the resin bottles 2 through the irradiation window 18 directly collides with the resin bottles 2 being conveyed at equal interval in front of the irradiation window 18 in the irradiation zone A, in one occasion, and on the other hand, in another occasion, the electron beam passes through a space between the adjacent resin bottles 2 and is trapped by the beam collector 46. When the electron beam irradiates the beam collector 46, the electrons in the beam flow toward the grounded earth and the electric current caused due the electrons is measured by the current measuring device 52. As shown in Fig. 3, such measured electric current value E1 is increased or decreased in accordance with a time period at which the electron beam is shut off by the collision with the resin bottles 2 and a time period at which the electron beam passes through the space between the resin bottles and directly irradiates the beam collector 46.

The electric current E1 measured by the electric current measuring device 52 is transmitted to the control device 42 and then compared with the reference value by the comparator 54. In such comparison, as mentioned hereinbefore, the current value becomes minimum at the time period at which the emitted electron beam is shout off by the resin bottles 2, and on the other hand, becomes maximum at the time period at which the electron beam passes through the space between the resin bottles 2.

Further, the judgement in the comparison of the measured electric current valve with the reference value is made based on the recognition such that a case in which the peak of the maximum or minimum value exceeds the upper limit reference value S1A, S1B is judged as "excess" and a case in which the peak thereof falls bellow the lower limit reference value S2A, S2B is judged as "shortage".

In the case where the electric current value shown with the solid line "E1" in Fig. 3 is measured, the peaks of the maximum and minimum values fall bellow the lower limit values S2A, S2B, and the electron beam amount for irradiating the resin bottle becomes insufficient, and hence, there is a fear such that the sterilization is not sufficiently performed and such resin bottle 2 is judged to be defective by the judging unit 56.

On the other hand, in the case where the electric current value shown with the broken line "E2" in Fig. 3 is measured, the peaks of the maximum and minimum values exceeds the upper limit values S1A. S1B, and the electron beam amount for irradiating the resin bottle becomes excessive, and hence, there is a fear such that the resin bottle 2 is deformed or discoloured and such resin bottle 2 is judged to be defective by the judging unit 56.

Further, in this judgement, since the minimum value has a low current value and a small deviation width, the judgement will be more easily made by monitoring the maximum value which is more remarkably changed. Furthermore, in a case where the irradiation amount of the electron beam from the electron beam irradiator 16 is constant, the resin bottle 2 is excessively irradiated in a case when the resin bottle 2 is conveyed at a slow speed, and on the other hand, the resin bottle is insufficiently irradiated in a case when the resin bottle 2 is conveyed at a high speed. Accordingly, the electron beam irradiation amount is controlled in response to the bottle conveying speed. In such control, the irradiation amount of the electron beam instructed by the electron beam irradiator 16 is recognized by measuring the electric current supplied to the electron beam irradiator 16 by a supply current recognition unit (current monitor) 60, and the electric current reference value is changed in accordance with such measured and recognized result.

The resin bottle 2 judged to be defective by the judging unit 56 of the control device 42 is specified by the pulse number from the encoder 40 provided for the rotary body 30 of the bottle conveyer 20. The resin bottle 2 specified is thereafter transferred to the vessel holder 34 of the discharge wheel 24 from the gripper 28 of the bottle conveyer 20. The discharge wheel 24 is also provided with the encoder 44, and the resin bottle 2 judged to be defective in the bottle conveyer 20 is traced after the transfer to the discharge wheel 24, and removed at the reject position B and discharged to the reject section 39. It is further to be noted that, in the present embodiment, not only the resin bottle 2 judged such that the measured electric current E1 is out of the reference value S1, S2 but also the resin bottles 2 positioned before and after this defective bottle 2 are pulled out together from the conveying line.

As mentioned above, the resin bottle 2, which is judged as being improper in irradiation amount of the electron beam based on the measured electric current value E1 from the beam collector 46 serving as the electric current measuring electrode, is taken out from the discharge wheel 24 and discharged to the reject section 39. On the other hand, the resin bottle 2, which is judged as being proper in irradiation amount of the electron beam, is transferred to the vessel holder 34 of the discharge wheel 24 from the gripper 28 of the bottle conveyer 20, and then further transferred to the vessel holder 38 of the carry-out wheel 36 provided in the subsequent intermediate chamber 35 so as to be subjected to the filling process, capping process and so on in the subsequent stage.

In the case where the current flowing from the beam collector 46 to the earth is measured by the current measuring device 52, and when the measured current values E1 and E2 vary largely as shown in Fig. 3, the judgement can be accurately made in comparison with the reference values S1A, S2A, S1B, S2B. However, as shown in Fig. 4, when the current values E3 and E4 increases or decreases by small amount, it is judged whether the irradiation amount of the electron beam is proper or not by obtaining average values C (C1, C2, C3, C4) in every predetermined interval (for example, every movement time, by one pitch, of the resin bottle 2 conveyed by the bottle conveyer 20) and comparing the average values C with the reference values (upper limit reference value S3 and lower limit reference value S4). In the case when the current value E3 shown in Fig. 4 with the solid line, the average value C3 is lower than the reference value S4, and hence, it is judged that the electron beam irradiation amount is insufficient. On the other hand, in the case when the current value E4 shown with the broken line is measured, the average value C4 exceeds the upper limit reference value S3, and hence, it is judged that the electron beam irradiation amount is excessive. Further, an interval shown by reference character P in Fig. 4 represents an interval corresponding to one pitch of the resin bottle 2.

According to the present embodiment of the structure and arrangement mentioned above, it becomes possible to detect a defective vessel and reject it in an early stage during the operation and to reduce, as much as possible, a fear of bringing contaminated substance such as virus and bacteria into downstream side environment. In a case even if a detective vessel arises, it can be found possibly immediately and appropriately handle such defective vessel, thereby preventing a lot of defective vessel from arising, thus being effective and advantageous.

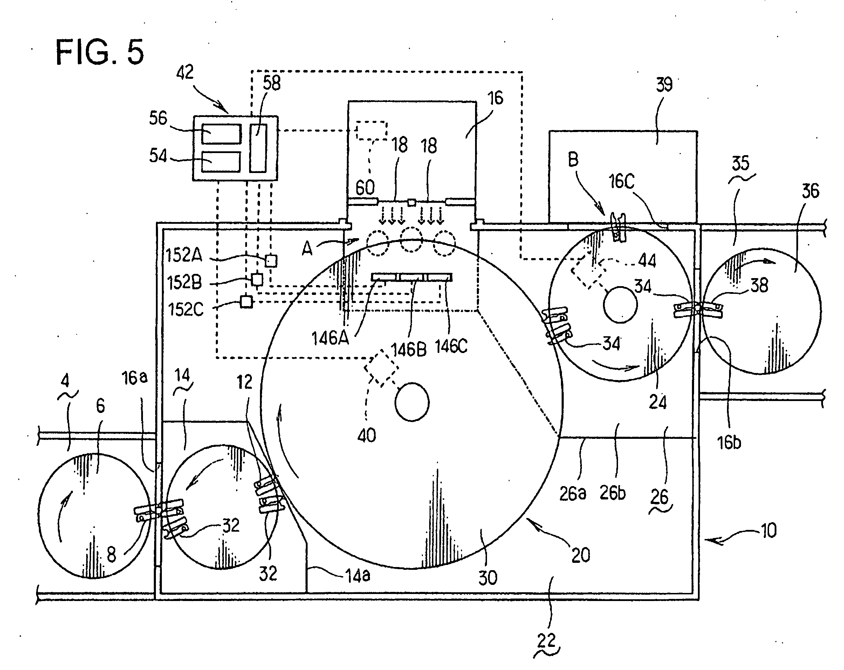

Fig. 5 shows an electron beam sterilizer according to a second embodiment of the present invention, which differs from the aforementioned first embodiment in the structure of the electric current measuring device for measuring the current of the electrode, i.e., electric current measuring electrode, and other structures and arrangements are substantially the same as those of the first embodiment shown in Fig. 1. Accordingly, like reference numerals are added to units or components corresponding to those of the first embodiment and description thereof is omitted herein and only different structures will be mentioned.

In the second embodiment shown in Fig. 5, a beam collector 146 is composed of divided three parts (146A, 146B, 146C), which are arranged in a manner insulated from each other, and these three beam collector parts 146A, 146B and 146C are disposed so as to correspond to three resin bottles 2 which are at once irradiated with the electron beam in the electron beam irradiating zone A, respectively.

Three electric current measuring devices 152A. 152B and 152C (152) are connected to the beam collector parts 146A, 146B and 146C, respectively, so as to independently measure the current values when the electron beam irradiation is performed. The respective beam collector parts 146A, 146B and 146C have heights higher than the height level of the irradiation window 18 and widths equal to or smaller than the width of the resin bottle 2. The reduction of the width of the beam collector parts contributes to easy comparison of the measured current values with the reference value because the current values measured by the current measuring devices 152A, 152B and 152C largely vary every time when the resin bottles 2 pass, and moreover, the resin bottles 2 which are irradiated insufficiently with the electron beam can be specified every one resin bottle 2.

## Claims

1. An electron beam sterilizer including: an electron beam irradiator (16) for irradiating a vessel (2) with an electron beam through an irradiation window (18) formed to the electron beam irradiator (16) ; and a vessel conveyer (20) for conveying the vessel (2), in which a vessel (2) conveyed by the vessel conveyer (20) in front of the irradiation window (18) is irradiated with the electron beam to thereby sterilize the vessel (2),
the electron beam sterilizer further comprising:
a comparator (54) that is arranged to compare a measured result from an electric current measuring means (52) with a predetermined reference value;
a judging means (56) that is arranged to judge whether an amount of the electron beam irradiated to the vessel (2) is proper or improper in accordance with the compared result;
a reject means that is arranged to take out a vessel from the vessel conveying path on a downstream side of an electron beam irradiating position; and
a command means (58) that is arranged to instruct take-out of a vessel (2) to the reject means based on the judgement result by the judging means (56); and
a supply current recognition means (60) that recognizes an electric current value to be supplied to the electron beam irradiator (16),
**characterized by**:
an electric current measuring electrode (46) being disposed so as to oppose to the irradiation window (18) with a vessel conveying path of the vessel conveyed by the conveyer (20) interposed therebetween, and further **characterized in that**:
the electric current measuring means (52) is arranged to measure an electric current value passing through the electric current measuring electrode (46) by the irradiation of the electron beam;
the electric current passing through the electric current measuring electrode (46) during the electron beam irradiation to the vessel (2) now being conveyed is measured, it is judged whether the electron beam irradiation amount to the vessel is proper or improper by the judging means, and a vessel (2), to which improper amount of the electron beam (56.) irradiation out of the predetermined reference value is applied, is rejected; and
the comparator (54) is configured to change the predetermined reference value in response to variation of the recognized supply electric current value.

2. The electron beam sterilizer according to claim 1, wherein the comparator (54) is configured to compare at least one of measured values of increasing electric current and decreasing electric current during the measurement thereof with the predetermined reference value, and the judging means (56) judges that when the measured electric current value is out of the predetermined reference, the electron beam irradiation amount is improper.

3. The electron beam sterilizer according to claim 1, wherein the comparator (54) is configured to obtain an average value of the electric current value increasing or decreasing during the measurement thereof as a measured result and compare the average value with the predetermined reference value, and the judging means (56) judges that when the average value is out of the predetermined reference, the electron beam irradiation amount is improper.

4. The electron beam sterilizer according to claim 1, further comprising a beam collector (46) which is disposed so as to oppose to the irradiation window (18) of the electron beam irradiator (16) with the vessel conveying path interposed therebetween, and the beam collector (46) has a size covering an entire area of the irradiation window (16) to thereby trap the electron beam and is grounded and supported in an electrically insulated state so as to be constructed as the electric current measuring electrode.

## Patentansprüche

1. Elektronenstrahl-Sterilisator mit: einem Elektronenstrahl-Bestrahler (16) zum Bestrahlen eines Gefäßes (2) mit einem Elektronenstrahl durch ein Bestrahlungsfenster (18), das an dem Elektronenstrahl-Bestrahler (16) ausgebildet ist, und mit einem Gefäßzuführer (20) zum Zuführen des Gefäßes (2), bei dem ein Gefäß (2), das durch den Gefäßzuführer (20) vor das Bestrahlungsfenster (18) zugeführt wird, mit dem Elektronenstrahl bestrahlt wird, um **dadurch** das Gefäß (2) zu sterilisieren,
der Elektronenstrahl-Sterilisator ferner mit:
einer Vergleichseinrichtung (54), die dafür angeordnet ist, ein von einer Messeinrichtung (52) für einen elektrischen Strom gemessenes Ergebnis mit einer vorher eingestellten Referenz zu vergleichen;
einer Beurteilungseinrichtung (56), die dafür angeordnet ist, gemäß dem Vergleichsergebnis zu beurteilen, ob eine Menge des Elektronenstrahls, der auf das Gefäß (2) bestrahlt wurde, korrekt oder inkorrekt ist;
einer Zurückweiseinrichtung, die dafür angeordnet ist, ein Gefäß von dem Gefäßzuführweg an einer zu einer Bestrahlungsposition des Elektronenstrahls nachgelagerten Seite zu entnehmen; und
einer Befehlseinrichtung (58), die angeordnet ist, um der Zurückweiseinrichtung ein Entnehmen eines Gefäßes (2) aufgrund des Beurteilungsergebnisses durch die Beurteilungseinrichtung (56) zu instruieren; und
einer Erfassungseinrichtung (60) für den Zufuhrstrom, die einen Wert des elektrischen Stroms erfasst, der zu dem Elektronenstrahl-Bestrahler (16) zugeführt wird, und
**gekennzeichnet durch**:
eine Messelektrode (46) für den elektrischen Strom, die so vorgesehen ist, dass sie dem Bestrahlungsfenster (18) mit einem Gefäßzuführweg des Gefäßes, das **durch** den Zuführer (20) zugeführt wird, dazwischen gegenüberliegt, und ferner **dadurch gekennzeichnet, dass**:
die Messeinrichtung (52) für den elektrischen Strom dafür angeordnet ist, einen Wert des elektrischen Stroms zu messen, der **durch** die Messelektrode (46) für den elektrischen Strom aufgrund des Bestrahlens **durch** den Elektronenstrahl hindurchfließt;
der elektrische Strom, der **durch** die Messelektrode (46) für den elektrischen Strom während der Elektronenstrahl-Bestrahlung des Gefäßes (2), welches gerade zugeführt wird, hindurchtritt, gemessen wird, es **durch** die Beurteilungseinrichtung beurteilt wird, ob die Menge an Elekronenstrahl-Bestrahlung auf das Gefäß korrekt oder inkorrekt ist, und ein Gefäß (2), auf das eine in Bezug auf die vorher festgelegte Referenz inkorrekte Menge an Bestrahlung **durch** den Elektronenstrahl(56) aufgebracht wird, zurückgewiesen wird; und
die Vergleichseinrichtung (54) dafür ausgestaltet ist, die vorher eingestellte Referenz entsprechend einer Veränderung in dem erfassten Wert des zugeführten elektrischen Stroms anzupassen.

2. Elektronenstrahl-Sterilisator nach Anspruch 1, bei dem die Vergleichseinrichtung (54) dafür ausgestaltet ist, wenigstens einen aus den gemessenen Werten des ansteigenden elektrischen Stroms und des abnehmenden elektrischen Stroms während dessen Messung mit der vorher festgelegten Referenz zu vergleichen und die Beurteilungseinrichtung (56) beurteilt, dass, wenn der gemessene Wert des elektrischen Stroms außerhalb der festgelegten Referenz liegt, die Menge an Elektronenstrahl-Bestrahlung inkorrekt ist.

3. Elektronenstrahl-Sterilisator nach Anspruch 1, bei dem die Vergleichseinrichtung (54) dafür ausgestaltet ist, einen Durchschnittswert des Werts des elektrischen Stroms, der während der Messung ansteigt oder abnimmt, als ein Messergebnis zu erhalten und den Durchschnittswert mit der vorher festgelegten Referenz zu vergleichen, und bei dem die Beurteilungseinrichtung (56) beurteilt, dass, wenn der Durchschnittswert außerhalb der vorher festgelegten Referenz liegt, die Elektronenstrahl-Bestrahlungsmenge inkorrekt ist.

4. Elektronenstrahl-Sterilisator nach Anspruch 1, ferner mit einem Strahlkollektor (46), der so vorgesehen ist, dass er dem Bestrahlungsfenster (18) des Elektronenstrahl-Bestrahlers (16) mit dem Gefäßzuführweg dazwischen gegenüberliegt, und der Strahlsammler (46) eine Größe aufweist, die eine gesamte Fläche des Bestrahlungsfensters (16) abdeckt, um somit den Elektronenstrahl einzufangen, und er geerdet und elektrisch isoliert so abgestützt ist, dass er als Messelektrode für den elektrischen Strom ausgebildet ist.

## Revendications

1. Stérilisateur par faisceau d'électrons comportant :
un irradiateur (16) à faisceau d'électrons pour irradier un récipient (2) avec un faisceau d'électrons à travers une fenêtre d'irradiation (18) formée pour l'irradiateur (16) à faisceau d'électrons, et un transporteur (20) de récipient pour transporter le récipient (2), dans lequel un récipient (2) transporté par le transporteur (20) de récipient en face de la fenêtre d'irradiation (18) est irradié par le faisceau d'électrons stérilisant ainsi la récipient (2),
le stérilisateur par faisceau d'électrons comprenant en outre :
un comparateur (54) qui est agencé pour comparer un résultat mesuré à partir d'un moyen de mesure du courant électrique (52) à une valeur de référence prédéterminée ;
un moyen d'estimation (56) qui est agencé pour estimer si une quantité du faisceau d'électrons irradiant le récipient (2) est appropriée ou non conformément au résultat comparé ;
un moyen de rejet qui est disposé pour éloigner un récipient du chemin de transport de récipient sur un côté en aval d'une position d'irradiation par faisceau d'électrons ; et
un moyen de commande (58) qui est disposé pour donner l'ordre au moyen de rejet d'éloigner le récipient (2) sur la base du résultat d'estimation par le moyen d'estimation (56) ; et
un moyen (60) de reconnaissance du courant d'alimentation, qui reconnaît une valeur de courant électrique à fournir à l'irradiateur (16) à faisceau d'électrons,
**caractérisé par** :
une électrode (46) de mesure du courant électrique disposée de manière à être opposée à la fenêtre d'irradiation (18) avec un chemin de transport de récipient pour le récipient transporté par le transporteur (20) interposé entre elles, et **caractérisé en outre en ce que** :
le moyen (52) de mesure du courant électrique est agencé pour mesurer une valeur de courant électrique traversant l'électrode (46) de mesure du courant électrique par l'irradiation du faisceau d'électrons ; et **en ce que**
le courant électrique traversant l'électrode (46) de mesure du courant électrique durant l'irradiation par le faisceau d'électrons du récipient (2) en cours de transport est mesuré, le moyen d'estimation estime si la quantité d'irradiation du récipient par le faisceau d'électrons est appropriée ou non, et un récipient (2), auquel une quantité inappropriée d'irradiation (56) au faisceau d'électrons en dehors de la valeur de référence prédéterminée est appliquée, est rejeté ; et **en ce que**
le comparateur (54) est configuré pour changer la valeur de référence prédéterminée en réponse à la variation de la valeur du courant électrique de l'alimentation reconnue.

2. Stérilisateur par faisceau d'électrons selon la revendication 1, dans lequel le comparateur (54) est configuré pour comparer au moins l'une des valeurs mesurées de l'augmentation du courant électrique et de la diminution du courant électrique pendant sa mesure avec la valeur de référence prédéterminée, et le moyen d'estimation (56) estime que lorsque la valeur mesurée du courant électrique est en dehors de la référence prédéterminée, la quantité d'irradiation au faisceau d'électrons est inappropriée.

3. Stérilisateur par faisceau d'électrons selon la revendication 1, dans lequel le comparateur (54) est configuré pour obtenir une valeur moyenne de l'augmentation ou de la diminution de la valeur du courant électrique durant sa mesure comme un résultat mesuré et pour comparer la valeur moyenne à la valeur de référence prédéterminée, et le moyen d'estimation (56) estime que lorsque la valeur moyenne est en dehors de la référence prédéterminée, la quantité d'irradiation au faisceau d'électrons est inappropriée.

4. Stérilisateur par faisceau d'électrons selon la revendication 1, comprenant en outre un collecteur de faisceau (46) qui est disposé de manière à s'opposer à la fenêtre d'irradiation (18) de l'irradiateur (16) à faisceau d'électrons avec le chemin de transport de récipient interposé entre eux, et la taille du collecteur de faisceau (46) couvre toute la superficie de la fenêtre d'irradiation (16) pour attraper ainsi le faisceau d'électrons, le collecteur est mis à la terre et supporté dans un état d'isolation électrique de façon à être construit comme étant l'électrode de mesure du courant électrique.
